# EUROPEAN PATENT APPLICATION

(11) **EP 1 716 886 A1**
(43) Date of publication of application: **02.11.2006**
(21) Application number: 04713093.5
(22) Date of filing: 20.02.2004
(51) Int. Cl.: A61N 5/073, A61N 5/067

(54) **EQUIPMENT AND METHOD FOR REDUCING AND ELIMINATING WRINKLES IN THE SKIN**

(71) Applicant: Arcusa Villacampa, Francisco Javier, E-08029 Barcelona (ES); Franco Lissott, Mailin, E-08029 Barcelona (ES); Cambier, Bernard, 08029 Barcelona (ES); Du Wulf, Anne Sophie, 08029 Barcelona (ES)
(72) Inventor: Arcusa Villacampa, Francisco Javier, E-08029 Barcelona (ES); Franco Lissott, Mailin, E-08029 Barcelona (ES); Cambier, Bernard, 08029 Barcelona (ES); Du Wulf, Anne Sophie, 08029 Barcelona (ES)
(74) Representative: Morgades y Manonelles, Juan Antonio
(86) International application number: PCT/ES2004/000046
(87) International publication number: WO 2005/079920

(57) **Abstract**

It includes means for preparing the area of the skin to be treated and means for applying energy thereto both being integrated in the same equipment. The means for preparing the area to be treated comprise an ultrasonic emitting device (preferably low frequency ultrasound) and/or dermabrasion device for breaking the lipid lamellar organization and reducing the dispersion of the irradiated energy altering the ultra-structure of the lipid corneal layer. Said means for applying energy comprise an emitting device capable to emit several combined forms of energy to the area to be treated. There is provided an atomizing device for applying topical agents and/or drugs to the area to be treated which cooperates with said means for, preparing the area to be treated.

The method provides emitting laser light and polarized light, intense pulsed light without polarization and polarized light, or intense pulsed light with polarization and polarized light.

## Description

The present invention relates to an equipment for reducing and/or removing wrinkles in the skin and to a method for carrying out said reduction and/or removal of wrinkles.

As it is well known, the skin is formed of three main layers. The outermost of said layers, the epidermis, has a surface layer (cornea) and an underlying layer which is involved in the formation of new elements as well as in determining of the colour of the skin. The dermis comprises the papillary layer and the reticular layer which has a structural function and it is formed by a series of intertwined fibrils where the collagen and the elastin are provided. Finally, the hypodermis, the innermost layer in the skin, is located under the dermis where blood vessels and nerves pass therethrough toward the epidermis.

The epidermis, in turn, is formed of four layers: the basal layer, the spinosum layer, the granulosum layer and the corneum layer. The "stratum corneum", the outermost layer in the skin, is the main barrier tissue preventing water loss of the body and it provides a mechanical, bacterial and chemical protection. This layer is formed of a continuous matrix of highly organized lipid lamellae inside of which an extensive network of corneocytes is embedded.

The corneocytes are plate like cells playing the crucial role in the mechanically resistant barrier tissue. These cells are located within the hydrophobic lipid lamellae.

By altering certain areas of the skin it is possible to modify the structure thereof for aesthetic purposes, for example for removing wrinkles. This alteration may take place by exposing the dermal layer of the skin to an appropriate energy radiation through the basal layer. This exposure produces that the collagen existing in the dermal layer undergoes a rise in temperature, while the basal layer remains intact to substantially inhibit the contact of the dermal layer with the environmental air.

The controlled radiation of an appropriate form of energy to the skin, e.g. light, gives rise to physical changes in the dermal layer inducing the thermal shrinkage of collagen structures.

In conventional techniques employed until recently, this phenomenon is achieved by removing a part or the whole epidermal layer (ablative technique) or by heating of the dermis without ablation (non ablative technique).

The present invention provides, by means of a new equipment which will be fully described hereinafter, reducing and/or removing wrinkles in the skin in a very effective way, since said equipment is able to induce the thermal shrinkage of the collagen without removing the spinosum layer and the basal layer.

One disadvantage of the prior art equipments is the fact that no polarized light is used in the post-treatment, which would help to speed up the neocollagen creation process.

The equipment of the invention is particularly, although not exclusively, intended to reduce and/or remove wrinkles in the skin. With said equipment it is possible to modify and/or induce structural changes in the skin and/or the subcutaneous tissue and it is particularly advantageous for treating developmental, environmental, aging, post-surgical, post-traumatic and certain aesthetic and pathological conditions.

The equipment comprises means for preparing the area of the skin to be treated and means for applying energy to said area to be treated.

The main feature of the equipment of the invention is that said means for preparing the area of the skin to be treated and said means for applying energy are all integrated in the same equipment.

More particularly, said means for preparing the area of the skin to be treated comprise an ultrasonic emitting device and/or dermabrasion device serving the purpose of breaking up the lipid lamellar organization and reducing the dispersion of the light irradiated to the area to be treated, as it will be disclosed in greater detail below, by altering the ultra-structure of the lipid corneal layer.

On the other hand, the means for applying energy comprise an emitting device which is able to emit either individually or in a combined way several types of energy to the area to be treated.

By emitting ultrasound and/or by dermabrasion it is possible to effectively break up the lipid lamellar organization to modify the absorption of light (coherent and/or non coherent) in the skin, and in particular to reduce the light dispersion. The lamellar lipid structures affect to the longer light wavelengths. By means of the application of ultrasonic and/or dermabrasion it is possible to alter the ultra-structure of the lipid corneal layer so that a better penetration of light or any other appropriate form of energy is thus obtained.

According to the invention, said ultrasonic emitting device is adapted to emit low frequency ultrasound to the area to be treated. Furthermore, in one embodiment, said device comprises an ultrasonic spatula provided with an end portion adapted to be arranged in contact with the area of the skin to be treated with the purpose of cleaning the surface layer of the epidermis, which has been previously impregnated by topical agents and/or appropriate drugs.

Application of low frequency ultrasound or dermabrasion an increased skin permeability is advantageously achieved (low frequency sonophoresis). This facilitates the penetration of such topical agents and/or drugs.

According to a further significant feature of the invention, the energy emitted to the area to be treated may be any of the forms of energy recited below:
- laser and polarized light
- intense pulsed light without polarization and polarized light; or
- intense pulsed light with polarization and polarized light.

Polarization is a state of radiant energy, more appreciable in the light, which vibrations take on a defined shape or direction as they are subjected to special influences. Light may be polarized by reflection, or by transmission, by means of polarizing optic elements.

Laser light (polarized coherent monochromatic light) has a clear effect on the cell metabolism. The response to the radiation may be altered in different ways through physical factors such as the wave length of the used source, or rising of the induced temperature.

The conventional equipments of the prior art do not combine laser light sources or intense pulsed light with a source of polarized light or IP²L.

The equipment of the present invention advantageously uses laser light or intense polarized pulsed light or non polarized intense pulsed light with the advantage that it allows to maintain the rising of temperature induced in the tissue during a larger period of time with regard to the prior art equipments.

In addition, the use of polarized light has the advantage that vasodilatation of the treated area is thus induced resulting in an increased transport of active substances to the cell.

Therefore, it is an advantage of the equipment object of the present invention the fact that, by using any of the previous light source combinations, and by exposing the area of the skin to be treated to a energy radiation combined with at least a source of laser light or polarized, intense pulsed light or non polarized, intense pulsed light, significant structural changes will be induced on the collagen matrix and on the fibroblast phenotype.

With the source of polarized light an increase in the amount of negatively charged sites on the fibroblast cell surface is obtained. This increase of negative surface charges may be considered as a biological effect of polarized light on the cell membrane. This would result in an increased membrane permeability. The action of the equipment that is herein described on the area to be treated causes releasing of growth factors of the fibroblasts stimulation as a consequence of a proliferative response of the fibroblast. This effect can not be achieved with non polarized light sources used by the conventional equipments.

The previously disclosed energy radiation is associated with the ultrasonic radiation as stated before as well as with the application of topical agents and/or drugs. This combination allows to efficiently improve the transcutaneously or transdermally administration of such active substances as their penetration is improved.

The equipment of the invention may be further provided with an atomizing device for applying said topical agents and/or drugs to the area to be treated. The atomizing device cooperates with said means for preparing the area to be treated.

According to the invention, a scanner may be also provided cooperating with said emitting device. Said scanner is adapted for automatically and evenly spreading the laser light beam incident on the area of the skin to be treated.

The invention also relates to a method for removing wrinkles in the skin which may be carried out by means of the previously disclosed equipment of the invention.

The method basically consists in impregnating the area of the skin to be treated with a topical agent and/or an appropriate drug, for example a cleaning substance for removing the surface dead cells in the epidermis. Ultrasound is subsequently applied by means of the above mentioned ultrasonic spatula of the equipment on the area to be treated which is impregnated with said topical agents and/or drugs. The area to be treated is then anesthetized and the ultrasonic spatula is applied again but from the reverse surface so that said topical agents and/or drugs are strongly and immediately absorbed by the area to be treated by sonophoresis. The following step consists in irradiating laser light, non polarized intense pulsed light or polarized intense pulsed light. This will allow a rise in temperature to be induced in the tissue during a certain period of time to cause significant structural changes in the collagen matrix and in the fibroblast phenotype, promoting vasodilatation of the treated area improving the transportation of metabolites and active substances to the cells.

Then the treated area is irradiated with polarized light in order to promote tissue recovery.

The features and the advantages of the equipment for removing wrinkles in the skin of the present invention will be readily understood from the detailed description of a preferred embodiment thereof which it will be given hereinafter by way of a non limitative example with reference to the drawings herein enclosed, in which:
Fig. 1 is a perspective view showing an example of an apparatus according to the invention;
Fig. 2 is an enlarged, side elevational view of the ultrasonic spatula used in the embodiment of the equipment of the invention shown in fig. 1;
Fig. 3 is an image obtained by light microscopy using polarized light corresponding to an area of the skin which has not been treated;
Fig. 4 is an image obtained by light microscopy using polarized light corresponding to an area of the skin which has been treated;
Fig. 5 is an image obtained by electron microscopy corresponding to the dermis treated by the equipment of the invention;
Fig. 6 is an image showing lysosomes to the cell surface;
Fig. 7 is an image showing the accumulation of substance around the fibroblast; and
Fig. 8 is an image showing large fibroblasts in the treated area.

The various references used in the description of the preferred embodiment of a device according to the present invention are listed below:
(1) equipment for removing wrinkles in the skin;
(2) display screen;
(3) control buttons;
(4) means for preparing the area to be treated;
(5) means for applying energy;
(6) ultrasonic spatula connecting cable;
(7) ultrasonic spatula;
(8) applicator of the means for applying energy which irradiates laser energy or polarized, intense pulsed light or non polarized, intense pulsed light;
(9) optical fiber cable of the means for applying energy: laser light conductor or polarized intense pulsed light conductor or non polarized intense pulsed light conductor;
(10) handle of the ultrasonic spatula;
(11) end portion for applying the ultrasonic spatula;
   (A, B) surfaces of the end portion of application of the ultrasonic spatula;
(12) atomizers;
(13) conduits of the atomizers;
(14) scanner for evenly spreading the laser energy;
(15) handpiece of the applicator laser light or of polarized intense pulsed light or non polarized intense pulsed light;
(16) lenses of the scanner; and
(17) polarized light lamp with mechanical arm.

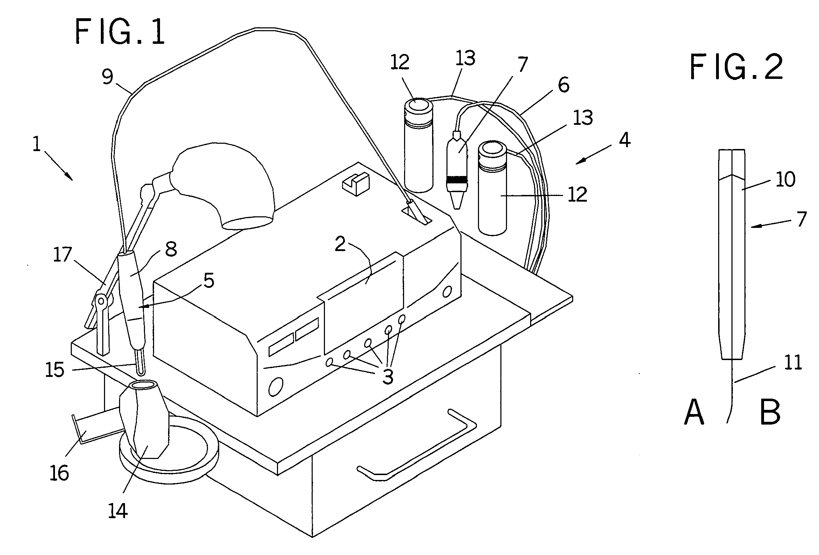

An example of an embodiment of a possible equipment for removing wrinkles in the skin according to the invention which has been generally indicated at (1) is shown in fig. 1.

The equipment (1) has the purpose of modifying and/or inducing structural changes on the patient's skin and/or subcutaneous tissue, with particular application in the treatment of developmental, environmental, aging, post-surgical, post-traumatic and certain aesthetic and pathological conditions.

Said equipment (1) comprises a display screen (2) to inform about various operating parameters of the equipment (1) which is controlled by front buttons (3).

Means for preparing the area to be treated (4) and means for applying energy (5) are provided, both being integrated in the equipment (1) itself, as shown in fig. 1.

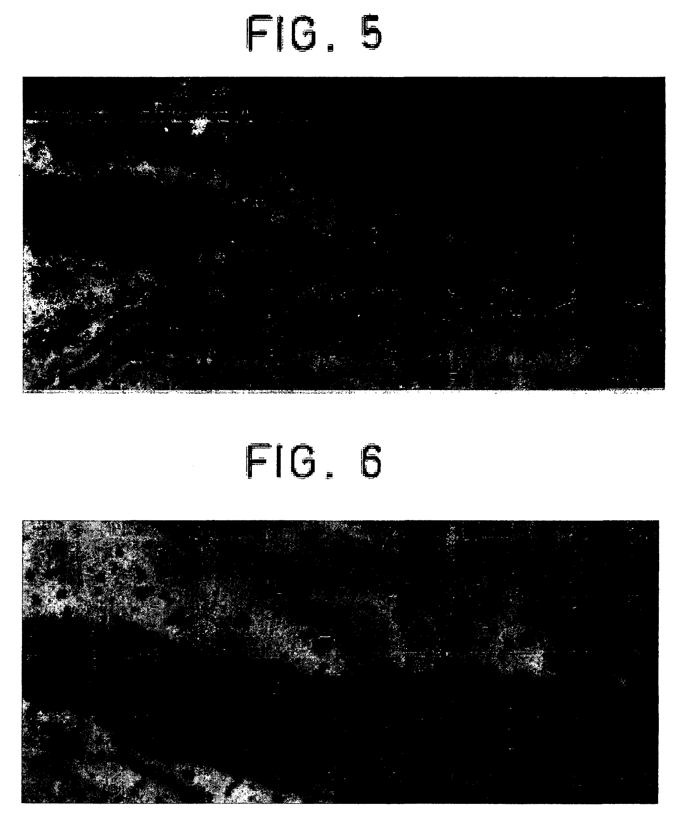

Said means for preparing the area to be treated (4) comprise an ultrasound emitting device which preferably is a low frequency ultrasound emitting device (15 KHz - 3 MHz) and/or a micro-dermabrasion mechanical device (0.01 - 3 bar) associated, by means of a cable (6), with an ultrasonic spatula (7), that may be seen more clearly from fig. 5 of the drawings herein attached.

As shown in said fig. 5, the ultrasonic spatula (7) is provided with a handle (10) and an applying end portion (11). Said end portion (11) is adapted to be arranged in contact with the area of the skin to be treated for cleaning the surface layer of the epidermis, which has been previously impregnated by topical agents and/or drugs.

Impregnation of the area of the skin to be treated is carried out by said means (4) of the equipment (1) for preparing the area to be treated. Said means (4) are also provided with atomizers (12) connected, through respective conduits (13), with a compressor. The compressor (not shown) operates a membrane and/or cylinder pneumatic device working at a pressure of the order of 0.1 - 3 bar. With the purpose of minimizing the vibration of the equipment (1) a double membrane pump may be used actuated by a single shaft and single motor.

The use of the ultrasonic spatula (7) of the equipment (1) allows for effectively breaking up the lipid lamellar organization of the surface layer of the skin. By applying the ultrasonic spatula (7) the layer of epidermis dead cells is therefore removed at the same time dispersion of the energy applied to the area to be treated by said means for applying energy (5) is reduced. This also allows improving the skin permeability by low frequency sonophoresis, as it will be detailed hereinafter, which facilitates delivering of topical agents and/or drugs as penetration thereof in the skin is improved.

The means for applying laser energy (5) comprise an applicator (8) that is connected, through an optical fiber cable (9), with an emitting device of the equipment (1). The applicator (8) transmits laser light, although it could be also, according to the invention, an intense pulsed light applicator with no polarization (IPL) or a polarized intense pulsed light (IP²L) applicator which would be formed by a flash lamp (Xenon or Krypton) and a polarizing filter in the case of IP²L and it would be connected with the equipment by means of electric cables and cooling conduits (air or water).

The means for applying wide spectrum polarized light includes a wide spectrum lamp (17), a reflector, a polarizing filter, a bandpass filter, and a mechanical arm.

One of these forms of energy is always a high power light source which may be either laser, IPL or IP²L, according to the invention. The other form of energy may be polarized light.

Here follows an example of values defining the energy emitted by the equipment (1) of the invention:
- laser light wave length: 400-2000 nm.
- wave length of intense pulsed light with no polarization: 400-2000 nm.
- wave length of intense pulsed light with polarization: 400-2000 nm.
- polarized light wave length 400-3000 nm.
- ultrasonic frequency: 15 KHz-3 MHz.

Using laser light or polarized, intense pulsed light or non polarized, intense pulsed light as an irradiated source of energy is possible to maintain the rise in temperature induced on the tissue during a considerable period of time. The use of polarized light also allows for effectively inducing the vasodilatation of the treated area, which improves the transportation of active substances to the cell, as noted above.

The equipment (1) is also provided with a scanner (14) adapted to be attached to the handpiece (15) of the applicator (8) of the laser emitting device. The scanner (14) is provided with lenses (16) intended to direct the energy emitted to the area of the skin to be treated. By means of the use of the scanner (14) an even and automatic irradiation is achieved, avoiding repeating application points, as it would occur in a hand operation only using the applicator (8) without said scanner (14). The hand operation may be useful, however, in patient's areas of difficult access.

The synthesis of the collagen in the human skin and the structural changes in the collagen network produced applying the equipment of the invention result in a significant reduction of wrinkles in the patient's skin.

The operation of the previously described equipment (1) is described below in connection with the enclosed figures.

The area of the patient's skin to be treated is first impregnated with an appropriate substance (topical agents, drugs, cleaning substance...) according to the process and the patient requirements by means of the atomizers (12) provided in the equipment (1).

Once the area of the skin to be treated has been impregnated with said this atomizers (12), the surface (A) of the end portion (11) of the ultrasonic spatula (7) of the equipment (1) is applied to said area of the skin by emitting low frequency ultrasound. This results in a dermal flow of the applied substance due to the pore formation in the skin by ultrasound. The combination of the ultrasonic emission with the application of said substance allows an effective removal of the surface dead cells from the patient's epidermis.

The area to be treated is then anesthetized and the ultrasonic spatula (7) is applied again but this time through the reverse surface (B) of said end portion (11). Therefore, the applied substance is strongly and immediately absorbed by the area to be treated by sonophoresis.

Through the applicator (8), which is attached to the scanner (14), if necessary, laser light or polarized intense pulsed light or non polarized intense pulsed light is emitted. This step has the purpose to cause a rise in temperature in the tissue during a given period of time resulting in significant structural changes in the collagen matrix and in the fibroblast phenotype. With said energy emission the vasodilatation of the treated area is also promoted thus improving the transportation of metabolites and active substances to the cells.

A further step is also provided consisting in irradiating the area to be treated with polarized light in the post-treatment to speed up the neocollagen creation process.

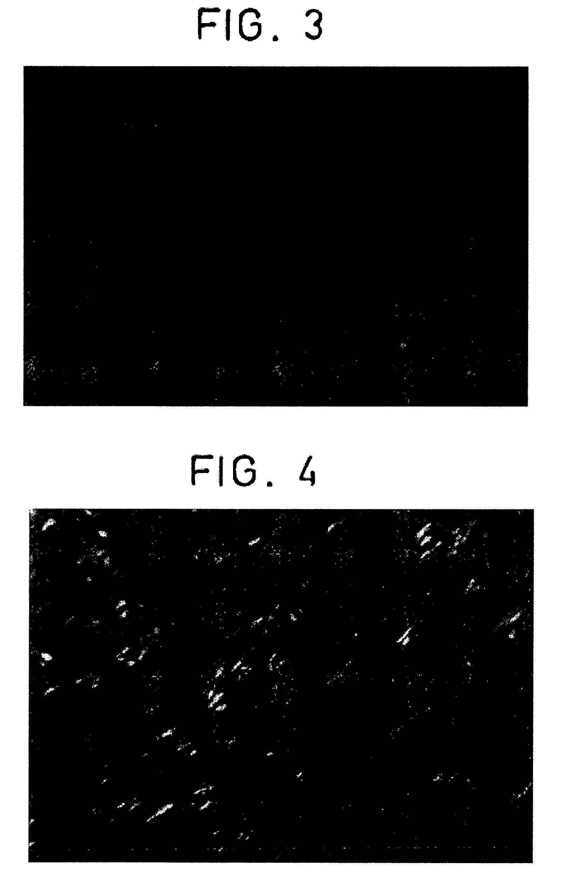

As it can be seen in the images of figs. 3 and 4 herein attached in the present specification, significant changes in the treated area (fig. 3) may be clearly seen through light microscopy caused in the collagen network after application of the equipment (1) of the invention as compared to the non treated area (fig. 4). Figs. 3 and 4 illustrate the ultra-structural changes in the collagen network in the human skin. In both figures the same microscope configuration has been used for the analysis. Comparing said figures it can be seen a significant increase of the collagen network polarization due to the structural changes on the fibrils organization.

These results were confirmed by means of electronic microscopy, as shown in the image of fig. 5 wherein beams are enlarged and/or the distance therebetween is changed. Changes in the distance between collagen network fibrils can explain the increased light reflection when observed through polarized light microscopy.

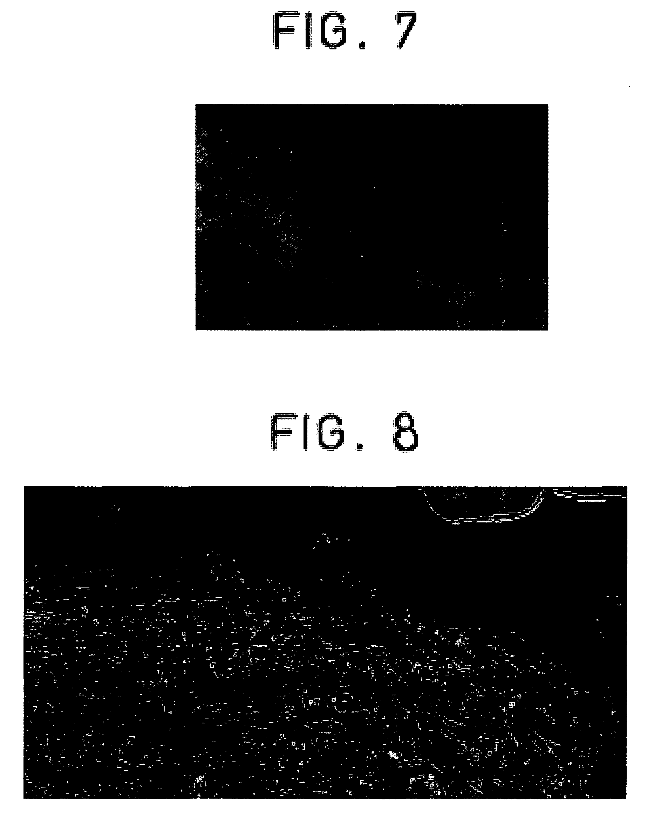

The fibroblasts are changed into a synthetic phase in which a tropocollagen active synthesis may be observed. The cell is enlarged and organules such as the endoplasmic reticulum and the Golgi apparatus are enlarged. According to fig. 6, lysosomes may be seen in the cell and multiple sites in the cell surface may be appreciated where tropocollagen is released.

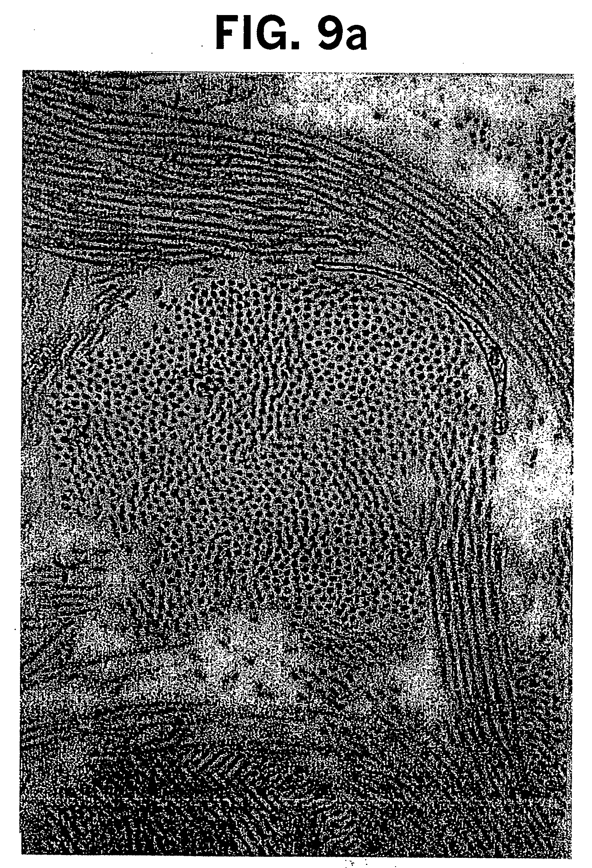

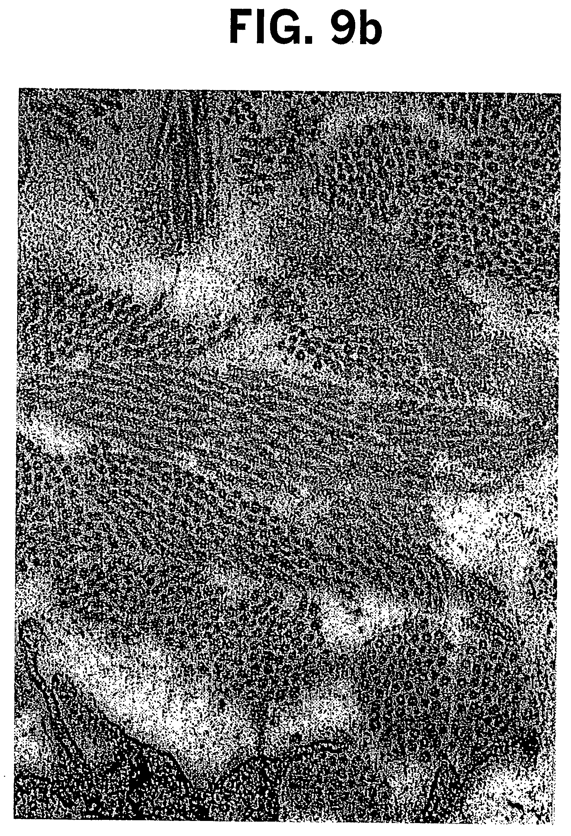

Particularly referring now to fig. 7 there is a significant substance accumulation around the cell as compared to a control. The fibroblast is in the synthetic phase. The cell increase may be also observed through light microscopy (fig. 8).

Once the equipment and the method for removing wrinkles in the skin of the present invention have been sufficiently described in connection with the enclosed drawings, it is understood that any detail modification may be introduced as appropriate, provided that variations may alter the essence of the invention as summarised in the appended claims.

## Claims

1. Equipment for reducing and/or removing wrinkles in the skin including means for preparing the area of the skin to be treated (4) and means for applying energy (5) to said area to be treated, **characterized in that** said means for preparing the area to be treated (4) and said means for applying energy (5) are integrated in the same equipment (1), said means for preparing the area to be treated (4) comprising an ultrasonic emitting device and/or dermabrasion device serving the purpose of breaking the lipid lamellar organization and reducing the dispersion of the irradiated energy altering the ultra-structure of the lipid corneal layer, and said means for applying energy (5) comprising an emitting device capable to emit in a combined way several forms of energy to the area to be treated.

2. Equipment for reducing and/or removing wrinkles in the skin according to claim 1, **characterized in that** said ultrasonic emitting device is adapted to emit low frequency ultrasound to the area to be treated.

3. Equipment for reducing and/or removing wrinkles in the skin according to claim 2, **characterized in that** said low frequency emitting device includes an ultrasonic spatula (7) adapted to be arranged in contact with the area of the skin to be treated with the purpose of cleaning the surface layer of the epidermis, which has been previously impregnated by topical agents and/or drugs.

4. Equipment for reducing and/or removing wrinkles in the skin according to claim 1, **characterized in that** the energy emitted to the area to be treated is laser light and polarized light.

5. Equipment for reducing and/or removing wrinkles in the skin according to claim 1, **characterized in** the energy emitted to the area to be treated is intense pulsed light without polarization and polarized light.

6. Equipment for reducing and/or removing wrinkles in the skin according to claim 1, **characterized in that** the energy emitted to the area to be treated is intense pulsed light with polarization and polarized light.

7. Equipment for reducing and/or removing wrinkles in the skin according to claim 4, **characterized in that** the wave length of the emitted laser light ranges from 500 to 2000 nm.

8. Equipment for reducing and/or removing wrinkles in the skin according to claim 5, **characterized in that** the wave length of the emitted pulsed light without polarization ranges from 400 to 2000 nm.

9. Equipment for reducing and/or removing wrinkles in the skin according to claim 6, **characterized in that** the wave length of the emitted intense pulsed light with polarization ranges from 400 to 2000 nm.

10. Equipment for reducing and/or removing wrinkles in the skin according to claim any of claims 4 to 6, **characterized in that** the wave length of the emitted polarized light ranges from 400 to 3000 nm.

11. Equipment for reducing and/or removing wrinkles in the skin according to claim 1, **characterized in that** the emitted ultrasonic frequency ranges from of 15 KHz to 3 MHz.

12. Equipment for reducing and/or removing wrinkles in the skin according to claim 1, **characterized in that** it further comprises an atomizing device (12) for the application of topical agents and/or drugs to the area to be treated which cooperates with said means for preparing the area to be treated (4).

13. Equipment for reducing and/or removing wrinkles in the skin according to claim 1, **characterized in that** it comprises a scanner (14) that cooperates with said emitting device and which is adapted to automatically and evenly spread the incident energy on the area of the skin to be treated.

14. Method for reducing and/or removing wrinkles in the skin, **characterized in that** it comprises the steps of:
- impregnating the epidermis with topical agents and/or drugs;
- applying ultrasound to the area to be treated impregnated with said topical agents and/or drugs with the purpose of eliminating the superficial dead cells;
- anesthetizing the area to be treated;
- inversely applying ultrasound to said area to be treated with regard to the way it was carried out previously so that said topical agents and/or drugs are strongly and immediately absorbed by the area to be treated through sonophoresis;
- irradiating laser light, or polarized, or non polarized, intense pulsed light to the area to be treated with the purpose of inducing a rise in temperature in the tissue during a certain period of time to cause significant structural changes in the collagen matrix and in the fibroblast phenotype and for promoting the vasodilatation of the treated area improving the transportation of metabolites and active substances to the cells.

15. Method for reducing and/or removing wrinkles in the skin according to claim 14, **characterized in that** it comprises the additional step of irradiating the area to be treated with polarized light in the post-treatment to speed up the neocollagen creation process.
